(19)

Europäisches
Patentamt
European
Patent Office
Office européen
des brevets

(11) **EP 1 055 392 B1**

(12) **EUROPEAN PATENT SPECIFICATION**

(45) Date of publication and mention
of the grant of the patent:
**19.03.2008 Bulletin 2008/12**

(51) Int Cl.:
*A61B 5/0215* (2006.01)

(21) Application number: **00850088.6**

(22) Date of filing: **18.05.2000**

(54) **Method for temperature compensation in a combined pressure and temperature sensor**

Verfahren zur Temperaturkompensation in einem kombinierten Druck- und Temperatursensor

Procédé de compensation de température dans un capteur combinant pression et température

(84) Designated Contracting States:
**AT BE CH CY DE DK ES FI FR GB GR IE IT LI LU MC NL PT SE**

(30) Priority: **27.05.1999 SE 9901962**
**27.05.1999 US 136401 P**

(43) Date of publication of application:
**29.11.2000 Bulletin 2000/48**

(73) Proprietor: **RADI MEDICAL SYSTEMS AB**
**754 50 Uppsala (SE)**

(72) Inventor: **Tulkki, Sauli**
**756 46 Uppsala (SE)**

(74) Representative: **Lindgren, Anders et al**
**BRANN AB**
**P.O. Box 1344**
**751 43 Uppsala (SE)**

(56) References cited:
**WO-A-90/06723          WO-A-98/42253**
**US-A- 4 554 927          US-A- 5 086 777**

- KO W H ET AL: "DEVELOPMENT OF A MINIATURE PRESSURE TRANSDUCER FOR BIOMEDICAL APPLICATIONS" IEEE TRANSACTIONS ON ELECTRON DEVICES, IEEE INC. NEW YORK, US, vol. ED-26, no. 12, 1 December 1979 (1979-12-01), pages 1896-1905, XP002039572 ISSN: 0018-9383

**Description**

The present invention relates generally to a method of determining the pressure, temperature and optionally flow in the medical area, especially to in situ measurement of the intracoronary pressure, distally of a stricture using a guide wire having a pressure sensor at its distal end.

**Background of the invention**

In order to determine and investigate the ability of a specific coronary vessel to supply blood to a heart muscle, i e the myocardium, there is a method through which the intracoronary pressure distally and proximal of a stricture are measured In the method the so called Fractional Flow Reserve (see "Fractional Flow Reserve" Circulation, vol. 92, No 11, December 1, 1995, by Nico H J. Pijls et al.). Briefly $FFR_{myo}$ is defined as the ratio between the pressure distally of a stricture and the pressure proximal of a stricture, i. e $FFR_{myo}=P_{dist}/P_{prox}$ The measurement of the distal pressure is made in the vessel using a micro-pressure transducer, and the proximal pressure is the arterial pressure .

In WO 97/27802 there is disclosed a sensor for temperature and pressure measurements having a pressure sensitive resistor (referred to as an active resistor) and a temperature sensitive resistor (referred to as a passive resistor). These resistors are part of a Wheatstone bridge comprising a total of four resistors and three "parasitic" cable resistances. Two different measurements are performed in this bridge, namely a measurement of a differential output voltage (potential difference between the Active and Passive circuit) which is used for pressure measurement and single ended output voltage (voltage across the Passive circuit), which is used for temperature measurement Only the passive and the active resistor are located on the actual sensor chip that is inserted into the body. The remaining resistors in the bridge are located externally, suitably in the control unit, and are coupled to the chip via electrical leads running inside a guide wire on the distal end of which the chip is located. This system functions appropriately when the environment is such that the properties of the electrical leads are not affected However, in view of the fact that the leads have extremely small dimensions and are relatively long, the resistance of the leads could vary considerably if the fraction of the length of the leads that are exposed to the body temperature varies, namely, if the guide wire need to be manipulated over distances of more than a few centimetres This effect is obtained in general when the temperature surrounding the guide wire changes e.g. when flushing with saline having a temperature differing the from body temperature These effects will have an impact on the temperature and pressure signals, and should be compensated for. In particular the read-out value of the temperature sensor (the passive resistor), which is also used for temperature compensation, will be incorrect Thus, it is desirable to obtain a correct temperature read-out One way of achieving this would be to provide separate leads coupled so as to enable a direct reading of the voltage drop across the passive (temperature sensitive) resistor. However, this would require the provision of five electrical leads inside the guide wire, and there is simply not space enough to make this possible.

In US-5,715,827 (Cardiometrics), there is disclosed apparatus for pressure measurements using two resistors provided on a flexible diaphragm The resistors are mounted so as to have opposite response to pressure, i e one resistor gives a positive signal and the other a negative. In the patent it is stated that this arrangement provides a temperature compensation for the signal. However, this arrangement has the same inherent problem with cable resistances affecting the signal

A method according to the preamble of claim 1 and a device according to the preamble of claim 12 are known from US-4,554,927.

**Summary of the invention**

The object of the invention is therefore to provide a measurement method using a single unit having as few electrical leads as possible, namely only three, and allowing compensation for variations in resistance of the leads coupling the resistors in the sensor to the control unit, in order to obtain a reliable measurement

The object outlined above is achieved according to the invention with the method of measurement defined in claim 1. The method comprises the excitation of the sensors so as to produce two distinguishable signals At least a component of at least one of the signals is representative of the cable resistance and can be used for compensation purposes

In a second aspect of the invention there is provided an apparatus suitable for such measurements having temperature compensation means The compensation means comprises circuitry for selectively and independently registering a resistance value for the passive resistor on the chip. The apparatus is defined in claim 12.

Further scope of application will become apparent from the detailed description given hereinafter

Nevertheless, it should be understood that the detailed description and specific examples, while describing a measurement method are given as an illustration only.

## Brief description of the drawings

The present invention will become more fully understood from the detailed description given herein below and accompanying drawings that are given as an illustration only, therefore the above description does not present any limitation to the invention at hand and herein

Fig. 1a shows a sensor/guide wire assembly to be used together with the invention,

Fig 1b shows a the distal tip of a catheter where a sensor is mounted and together with which the invention is to be used;

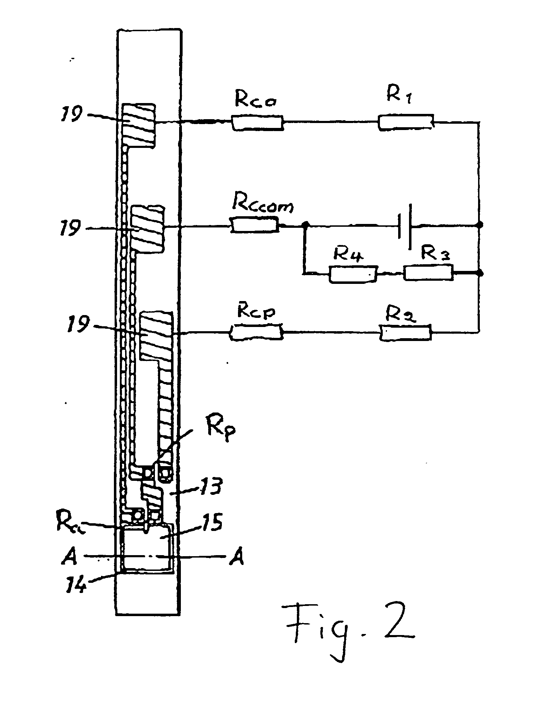

Fig. 2 is a schematic top view of a sensor chip and the resistances in the system

Fig. 3 is a simplified schematic circuit of the system used, without the inventive feature,

Fig 4 is a simplified schematic circuit of the system used, with a first embodiment of the inventive feature,

Fig 5 is a simplified schematic circuit of the system used, with a second embodiment of the inventive feature;

Fig 6 shows an equivalent circuitry of the circuitry in Fig 5 from a DC point of view,

Fig. 7 is a simplified schematic circuit showing an alternative configuration of the measurement system with the inventive feature;

Fig 8 is a graph showing the improvement in temperature readout with compensation according to the invention compared to without compensation

## Detailed description of the system and the method of measurement

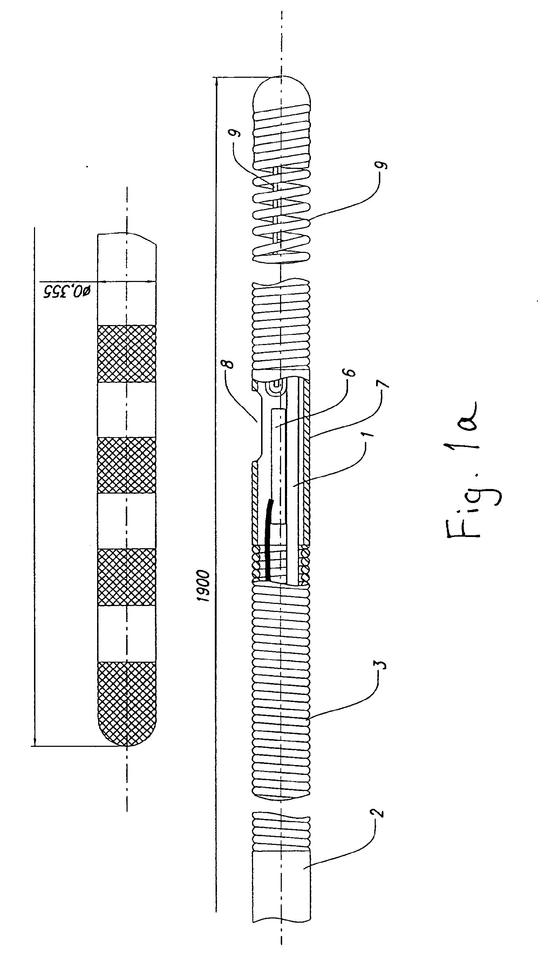

A distal portion of a sensor and guide wire assembly as disclosed in WO 97/27802, is shown in Fig 1a. The device comprises a solid wire 1 inserted into a proximal tube portion 2 The wire 1 forms the distal portion of the guide wire and is extended beyond the distal end of the proximal tube 2 where the tube is connected to, or provided with an integrally formed, spiral portion 3 At the distal end of the wire 1, a pressure sensor 6 is mounted Between the wire 1 and the spiral portion 3, electrical leads from the electrical circuitry run parallel with the wire 1. The sensor 6 is protected by a short section of a tube 7 having an aperture 8 through which a surrounding medium acts on the pressure sensor At the very distal end of the device there is a radio opaque coil 9 made of Pt and used for location purpose There is also provided a safety wire 10 for securing the distal part or the spiral 9.

The proximal tube 2 and the spiral portion 3 may be connected in such a way that they can be used as electrical shield.

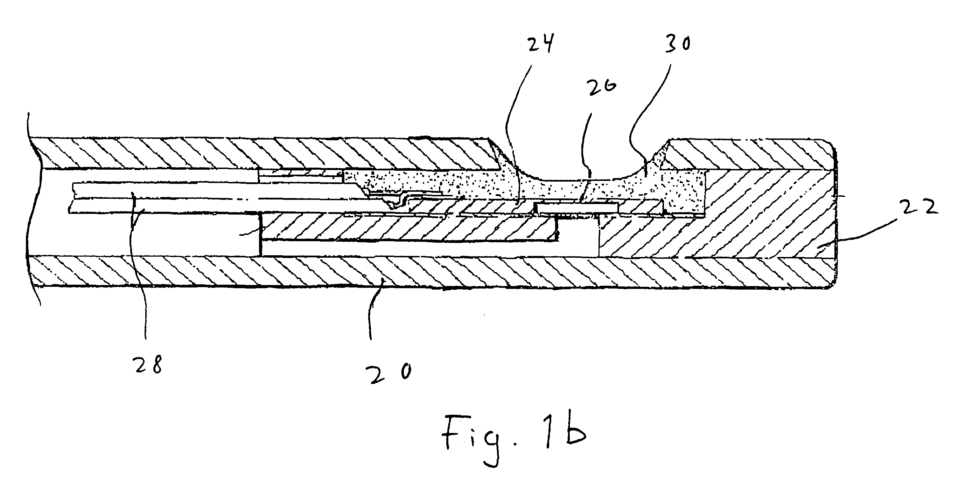

In Fig 1b there is shown a catheter tip pressure transducer which can be used with th present invention. This system comprises a catheter 20 closed by an end member 22 This member also functions as a support for a sensor 24 having a pressure sensitive diaphragm 26. Electrical leads 28 couple the sensor to external equipment The sensor may be protected by a material such as silicon rubber 30.

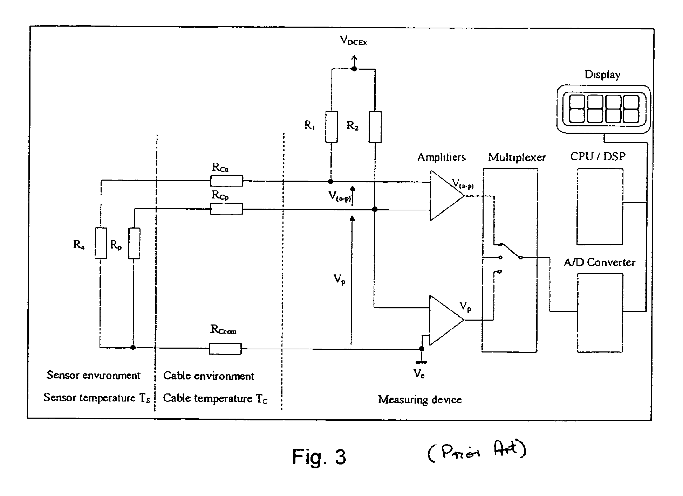

The prior art measurement system disclosed in WO 97/27802 will now be described with reference to Fig. 3

Pressure sensing resistor $R_a$ (a for "active") and temperature sensing resistors $R_p$ (p for "passive") are located on the actual sensor chip, and are coupled to the measurement device using insulated cables having resistances $R_{ca}$, $R_{cp}$ and $R_{ccom}$, these cables are mounted inside said proximal tube. These cables couples resistors $R_a$, $R_p$ into the measurement device, and together with internal resistors $R_1$ and $R_2$ they form a Wheatstone bridge. This Wheatstone bridge is driven by the excitation voltage $V_{DCEx}$, and the differential output voltage $V_{(a-p)}$ from the bridge is read using an instrumentation amplifier. Single ended output voltage $V_p$ in the temperature sensing circuit is read by an other instrumentation amplifier Amplifier output signals $V_{(a-p)}$ and $V_p$ are coupled into the multiplexer, the amplifier outputs $V_{(a-p)}$ and $V_p$ being sequentially switched into an A/D converter for conversion into digital data This data is read from A/D converter using a microcontroller CPU or digital signal processor DSP CPU/DSP performs calculations needed to obtain the final pressure or temperature signal, and finally the measured values are presented on a numerical display

The prior art measurement method will now be described with reference to Fig. 3

$R_a$ and $R_p$ are the resistances of the active sensor element and the resistance of the passive element respectively, as previously described Their resistance may vary differently with the temperature i.e. $TC_{Ra} \neq TC_{Rp}$. Therefore a compensation of the pressure signal has to be made to compensate for the error related to varying temperature, i e.

it is known from the calibration curve which is achieved during manufacturing process how much has to be added to or subtracted from the registered pressure signal in order to get a correct pressure value

During pressure measurement the circuit disclosed in Fig. 3 is supplied with excitation voltage or current (DC or AC). At present most systems of the type the present invention is concerned with are operated with excitation voltages of up to 15 V. However, it is conceivable to operate at substantially higher voltages, provided the components of the system are designed accordingly The differential voltage the active and the passive circuits $V_{(a-p)}$ is a representative signal for the measured pressure, and single ended voltage $V_p$ is a representative signal for the measured temperature. Because of the fact that the resistors $R_a$ and $R_p$ are not identical in terms of their temperature dependence, the differential voltage $V_{(a-p)}$ will be temperature dependent, therefore the pressure signal $V_{(a-p)}$ has to be compensated against varying temperature During the manufacturing process every sensor is characterised in terms of temperature dependence and the calibration parameter is achieved by measuring simultaneously the temperature coefficient $TC_{Rp}$ of the passive element and the difference between $TC_{Rp}$ and temperature coefficient $TC_{Ra}$ of the active sensing element, this is done during manufacturing process The result is thus a compensation factor

$$T_{Factor} = \frac{TC_{Ra} - TC_{Rp}}{TC_{Rp}} \qquad (10)$$

which is used to compensate the sensor signal for temperature variations This factor which is unique to each individual sensor, is preferably stored in a PROM or the like, attached to the assembly of which the sensor forms a part. The compensated sensor signal is then equal to

$$V_{a-p} - T_{Factor} * V_P \qquad (11)$$

where, $V_{a-p}$ and $V_p$ are the measured potential difference between the active and the passive circuit, and the measured potential drop across the passive circuit, respectively (see Fig. 3).

However, as indicated previously, this method has a drawback which has to do with the potential $V_p$ being dependent on changes of the cable resistance as well, (i.e $R_{Cp}$, and $R_{Ccom}$), such that

$$V_p = V_{Ex} \frac{R_{Cp} + R_P + 2R_{C\,com}}{R_{Cp} + R_P + 2R_{C\,com} + R_2} \qquad (12)$$

where, $V_{Ex}$ is the excitation voltage of the bridge (this is a simplification under the previously made assumptions).

The prior art measurement method will function satisfactorily provided that the signal produced by change in the resistances of the cable, i e $R_{Ca}$, $R_{Ca}$ and $R_{Ccom}$ due to the temperature, is negligible compared to the measured temperature signal However, when e g small dimensions is needed the resistance per unit length becomes high, which results in substantial total resistance of the cable In order to illustrate this effect the following example is given The cables used in the above mentioned International Patent Application WO 97/27802 have a diameter 0 050 mm, length 1750 mm, resistance ≈ 40 ohm The temperature coefficient of copper is ≈ 0 00433 ˚C$^{-1}$, which results in an increase of resistance in the cable by 0 173 ohm/˚C. The effective influence of this resistance change in the passive circuit used for temperature measurement is three times higher because of the fact that the total length of cable in the circuit is 2 * 1750 mm ($R_{Cp}$ + $R_{Ccom}$) and the fact that current passing trough the common cable $R_{Ccom}$ is a sum of currents in the passive and active circuit, the effective influence of the cable can now be defined as 0 520 ohm/˚C. Now, if the temperature difference between the room temperature and the body temperature is approximately 17 ˚C resulting in the resistance change of 8.33 ohm if the hole cable length is moved from room temperature into body temperature This shall be compared with the fact that the temperature coefficient of the passive resistor $R_p$ located on the sensor chip is typically ≈ 0 000300 ˚C$^{-1}$ The resistance of the named resistor is typically ≈ 3000 ohm, which results in the desired temperature signal of 0 9 ohm/˚C. The relationship between undesired change in the cable resistance and desired temperature signal can now be calculated as 0 52 / 0 9 ≈ 60 %, which means that 37 % of the temperature signal can actually originate from the change in cable temperature. This fact is crucial especially when the sensor is used for the temperature measurements, the temperature compensation of the pressure measurements are also influenced Namely, there are applications where the cable length, which is exposed to the temperature change during the measurement is almost impossible to predict prior to the measurement procedure. The parts of the cable that will undergo a resistance change are those that are present in the same environment as the active sensor element (i.e. exposed to body temperature, higher than ambient) The contribution to the signal from the change of the cable resistances will thus be dependent on that length In case the length affected by the temperature change is large, the contribution will also be large Thus, a compensation for the change of the cable resistance is necessary

In another prior art measurement method and system disclosed in the above mentioned US-5,715,827 there are provided two "active" resistors, coupled in a first and a second branch of a Wheatstone bridge respectively In this system the resistors have opposite response to pressure, such that one of the resistors responds positively to pressure changes and the other responds negatively to a pressure change. However, the basic inherent problem with "parasitic" cable or lead resistances is the same as for the above discussed system. The present invention is equally well applicable to the later system without inventive skills being required

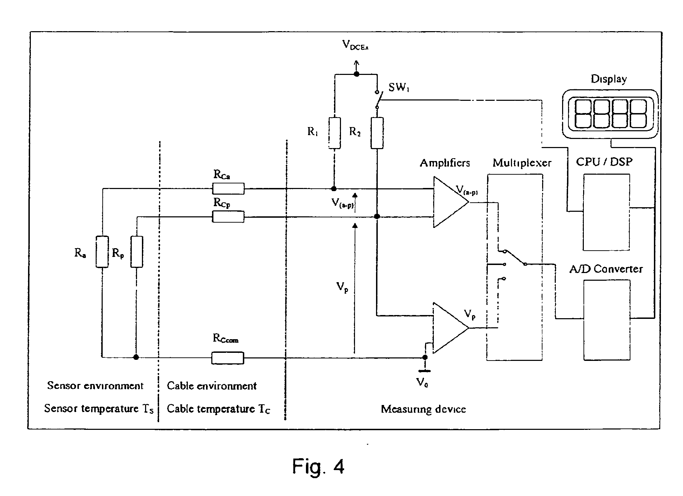

Now a first embodiment of the apparatus according to the invention will be described with reference to Fig. 4.

Basically the same set up as in the prior art measurement system is used However, there is the additional provision of means for providing two in time differing "modes" of excitation, which are generated by the a $SW_1$.

Switch $SW_1$ is coupled between $V_{DCEx}$ and $R_2$ in order to cut the excitation voltage to the passive circuit. If a measurement is now carried out with $SW_1$ open, and if one assumes that sum of $R_p$ and $R_{Cp}$ is negligible compared to the input impedance of the amplifiers that measures the voltages $V_p$ and $V_{a-p}$, $V_p$ will then be a measure of potential drops across $R_{Ccom}$ (no current is flowing in the passive circuit), i e the contribution to the measured temperature signal from the change of the cable resistances due to the temperature. Now that the contribution to the temperature signal from the change of the cable resistances is determined it is possible to obtain the compensated temperature signal. It is given by

$$V_p - 3 * V_{Popen} \qquad\qquad (1\ 4)$$

where, $3*V_{P\ open}$ is the compensation for the change of the cable resistances and $V_{Popen}$ is the voltage measured across the passive circuit with $SW_1$ open.

When $SW_1$ is closed the active element of the sensor yields a signal representative of the pressure, and its value is given by the signal $V_{a-p}$. The switch $SW_1$ is operable at a selected frequency, the magnitude of which is dependent on other system parameters, and could vary within wide limits

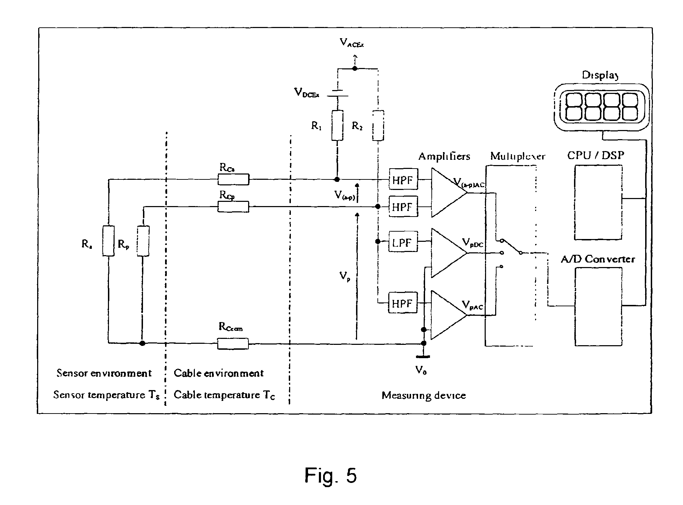

Now a second embodiment of the apparatus according to the invention will be described with reference to Figs 5-6

Another way of obtaining a compensation for the cable resistance is to create two different modes of excitation using two different excitation voltages separated from each other in frequency. A schematic illustration is shown in Fig 5.

In this embodiment the Wheatstone bridge is driven by the excitation voltage $V_{ACEx}$ which is common to Active and Passive circuit There is also a second excitation mode implemented, in this example a DC-voltage It could of course be an AC voltage of a different frequency or phase This additional DC excitation is applied in the Active circuit Now there are three signals or components of signals which are of interest in this application, the AC-component of the differential voltage $V_{(a-p)}$ referred to as $V_{(a-p)AC}$ in Fig 5, which is a measure of the pressure, the AC-component of the single ended voltage $V_p$ in Fig 5 refereed to as $V_{pAC}$ , which is a measure of the temperature, and finally DC-component of the single ended voltage $V_p$ in Fig. 5 referred to as $V_{pDC}$ , which is proportional to the resistance in the common cable $R_{Ccom}$

In order to obtain the different components of the differential voltage $V_{(a-p)}$ and the single ended voltage $V_p$ it becomes necessary to employ filtering functions.

The AC-component of the differential voltage $V_{(a-p)}$ is obtained by high-pass filtering the signal before entering into the amplifier The output from this amplifier is then fed into the multiplexer and further to the A/D converter where the signal is converted into the digital code. This digital code is read by CPU/DSP which also calculates e.g. the RMS (root mean square) value of the pressure signal.

The AC-component of the single ended voltage $V_p$ is obtained by high-pass filtering the signal before entering into the amplifier. The output from this amplifier is then fed into the multiplexer and further to the A/D converter where the signal is converted into the digital code This digital code is read by CPU/DSP which also calculates e.g. the RMS (root mean square) value of the temperature signal.

An additional amplifier is introduced to obtain the DC-component of the single ended voltage $V_p$ which is also low-pass filtered before entering said amplifier. The output from this amplifier is then fed into the multiplexer and further to the A/D converter where the signal is converted into the digital code, this digital code is read by CPU/DSP

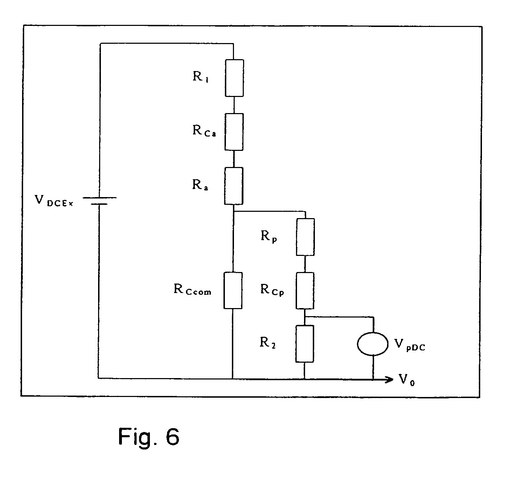

It is reasonable to assume that the internal impedance of the AC excitation source is near zero Under this assumption we can draw the equivalent circuit for calculation of the $R_{Ccom}$ which is shown in the Fig 6

In order to simplify the algorithm we can assume that $R_{Ccom} = R_{Cp} = P_{Ca} << R_1 = R_2 = R_a = R_p$

Now we will be able to calculate the resistance in the common cable It is given by

$$2 * V_{pDC} * ( R_1 + R_a ) / (V_{DCEx} - 2 * V_{pDC})$$

It is also possible to obtain the compensated temperature signal It is given by

$$V_{pAC} - 3 * V_{pDC}$$

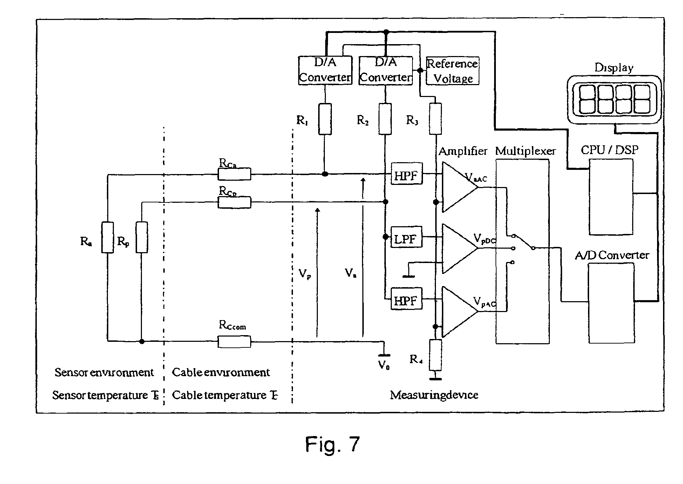

Now an alternative implementation of the second embodiment of the invention will be described with reference to Fig. 7

The control unit shown in Fig. 7, comprises a first and a second instrumental amplifier having inputs for receiving said temperature signal respectively, and outputs delivering a processable out-signal. It comprises a multiplexer coupled to the outputs of said amplifiers, an A/D converter coupled to said multiplexer, and a control unit coupled to sequentially receive outputs from said A/D converter.

It is provided with excitation means comprising a DC voltage source and an AC voltage source, whereby the DC and AC excitation voltages are superimposed on each other

The control unit further comprises high pass filter means coupled to the input of said first instrumental amplifier, said input being coupled so as to receive a high frequency component of said pressure signal. The second instrumental amplifier also has a low pass filter means coupled to its input, said input being coupled so as to receive a low frequency component of said temperature signal The third instrumental amplifier has a high pass filter means coupled to its input, said input being coupled so as to receive a high frequency component of said temperature signal and outputs delivering a processable out-signal Furthermore there is a multiplexer coupled to the outputs of said amplifiers, an A/D converter coupled to said multiplexer, and a control unit coupled to sequentially receive outputs from said A/D converter

In this alternative implementation the Wheatstone bridge is driven by two D/A converters controlled from the CPU/DSP unit. There is also a reference voltage source which is used for the setting of the full scale output from the D/A converters. This reference is also fed into the voltage divider network containing resistors $R_3$ and $R_4$, the middle point voltage from this divider is used as reference level for the input amplifiers measuring $V_{aAC}$ and $V_{pAC}$, this makes it possible to use higher gain without saturating these amplifiers.

Two excitation modes can now be produced by supplying D/A converters with samples of data i e D/A converter supplying the circuit containing $R_1$ $R_{ca}$ and $R_a$ is fed with samples containing the waveform used as common excitation superimposed to the DC level used as second excitation mode, the D/A converter supplying the circuit containing $R_2$ $R_{Cp}$ and $R_p$ is fed with samples containing only the waveform used as the common excitation

Now there are three signals or components of signals which are of interest in this application the AC-component of the single ended voltage $V_a$ referred to as $V_{aAC}$ in Fig 7, the AC-component of the single ended voltage $V_p$ in Fig 7 referred to as $V_{pAC}$ , which is a measure of the temperature, the pressure is calculated as difference between $V_{aAC}$ and $V_{pAC}$ and finally the DC-component of the single ended voltage $V_p$ in Fig. 7 referred to as $V_{pDC}$ , which is proportional to the resistance in the common cable $R_{Ccom}$.

In order to obtain the different components of the voltages $V_a$ and $V_p$ it becomes necessary to implement filtering functions.

In this implementation the AC-component of the single ended voltage $V_a$ is obtained by high-pass filtering the signal before feeding it into the amplifier The output from this amplifier is then fed into the multiplexer and further to the A/D converter where the signal is converted into the digital data This digital data is read by CPU/DSP which also calculates e g the RMS (root mean square) value of the signal

The AC-component of the single ended voltage $V_p$ is obtained by high-pass filtering the signal before entering into the amplifier. The output from this amplifier is then fed into the multiplexer and further to the A/D converter where the signal is converted into the digital data This digital data is read by CPU/DSP which also calculates e.g. the RMS (root mean square) value of the temperature signal

An additional amplifier is introduced to obtain the DC-component of the single ended voltage $V_p$ which is also low-pass filtered before entering said amplifier. The output from this amplifier is then fed into the multiplexer and further to the A/D converter where the signal is converted into the digital data, this digital data is read by CPU/DSP.

The same algorithms as for the fig 5 yields and can be used to achieve the cable resistance and the compensated temperature signal

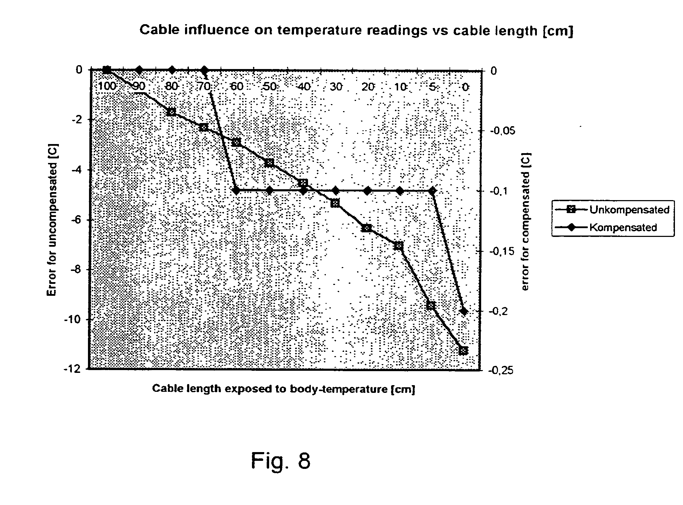

In order to illustrate the effect of the compensation obtained using apparatus in accordance with invention the following example is given The measurements shown in the diagram Fig 8 are achieved using the sensor disclosed in WO 97/27802 At the beginning of the experiment the sensor and 100 cm of the cables are immersed in the tank with 37 ˚C water, the rest of the cable (75 cm) and measuring unit is located in the room temperature $\approx$ 20 ˚C. Measured values $V_p$ of the uncompensated and compensated temperature signals are recorded for every 5 cm while the cable is removed from the tank. This experiment clearly demonstrates that the error of the uncompensated signal which is about 12 ˚C is decreased to less than 0.2 ˚C in the compensated signal

**Claims**

1. A method of selectively measuring pressure and/or temperature in vivo, comprising the steps of
providing a pressure sensor having two piezoresistive elements ($R_a$, $R_p$), said piezoresistive elements being coupled to a control unit by cables ($R_{C_{com}}$, $R_{C_a}$, $R_{C_p}$) having a temperature dependence affecting the signal obtained from said sensors,
providing excitation to said sensor to yield two different signals therefrom, such that at least a component of at least one of said signals is representative of the cable resistance, and **characterised by** the step of
using said quantity representative of the cable resistance value to compensate for the influence of said cable resistance on the at least one of said temperature and pressure signals.

2. The method according to claim 1, wherein the first piezoresistive element ($R_a$) is part of one branch of a Wheatstone bridge, and the second piezoresistive element ($R_p$) is part of another branch of said Wheatstone bridge, and wherein the excitation comprise a first excitation of the entire bridge and a second excitation of only the branch of the bridge containing the first piezoresistive element.

3. The method according to claim 2, wherein the excitation is a DC voltage applied to the bridge

4. The method according to claim 3, wherein the second excitation is carried out by switching off the branch containing the second resistor.

5. The method according to claim 4, wherein said second excitation is performed at a lower frequency than the first excitation

6. The method according to claim 1, wherein the excitation constitutes a composite excitation at two different frequencies

7. The method according to claim 6, wherein said composite excitation comprises a DC voltage excitation and an AC voltage excitation.

8. The method according to claim 1, wherein the excitation voltage is up to 15 V

9. The method according to claim 6, wherein the AC-component of the signal from the first sensor is obtained by high-pass filtering the signal before feeding it into an amplifier; the output from said amplifier is then fed into a multiplexer and further to an A/D converter where the signal is converted into digital data; said digital data is then read by a CPU/DSP which calculates e.g the RMS (root mean square) value of the signal, the AC-component of the signal from the second sensor is obtained by high-pass filtering the signal before feeding it into said amplifier, the output from said amplifier is then fed into said multiplexer and further to said A/D converter where the signal is converted into digital data, said digital data is then read by a CPU/DSP which also calculates e.g. the RMS (root mean square) value of the temperature signal, and wherein the DC-component of the signal from the second sensor is low-pass filtered, and said filtered signal is fed into a second amplifier, the output from said second amplifier being fed into said multiplexer and further to said A/D converter where the signal is converted into digital data, this digital data being read by CPU/DSP.

10. The method according to claim 1, wherein the sensor is mounted on a guide wire

11. The method according to claim 1, wherein the sensor is mounted on a catheter

12. A control unit in combination with a guide wire assembly, the control unit being suitable for processing signals from a sensor said assembly being adapted to selectively measure pressure and/or temperature in vivo, said guide wire assembly comprising a pressure sensor and electrical conductors ($R_{C_{com}}$, $R_{C_p}$, $R_{C_a}$) coupling said sensor to said control unit, said sensor having two piezoresistive elements ($R_a$, $R_p$), said assembly comprising parasitic resistances

($R_{C_{com}}$, $R_{C_p}$, $R_{C_a}$) in said electrical conductors coupling said sensor to the control unit, the control unit comprising means for the selective excitation of said pressure sensor ($SW_1$) so as to yield two different signals therefrom, means for the selective registering of a component of one of said signals generated m response to excitation of said sensor, said component being proportional to said parasitic conductor resistances, and

**characterised by**

means for compensating at least one of said different signals for the influence of said parasitic resistances, by using said component representative of the parasitic resistance value.

13. The control unit as claimed in claim 12, wherein the excitation means comprises a DC voltage source.

14. The control unit as claimed in claim 13, comprising a switch ($SW_1$) operable so as to enable cutting the excitation voltage to the passive circuit at a selected frequency

15. The control unit as claimed in claim 14, comprising a first and a second instrumental amplifier having inputs for receiving said temperature signal respectively, and outputs delivering a processable out-signal, a multiplexer coupled to the outputs of said amplifiers, an A/D converter coupled to said multiplexer, and a control unit coupled to sequentially receive outputs from said A/D converter

16. The control unit as claimed in claim 12, wherein the excitation means comprises a DC voltage source and an AC voltage source, whereby the DC and AC excitation voltages are superimposed on each other.

17. The control unit as claimed in claim 15, comprising a first instrumental amplifier having high pass filter means coupled to its input, said input being coupled so as to receive a high frequency component of said pressure signal, a second instrumental amplifier having a low pass filter means coupled to its input, said input being coupled so as to receive a low frequency component of said temperature signal, and a third instrumental amplifier having a high pass filter means coupled to its input, said input being coupled so as to receive a high frequency component of said temperature signal and outputs delivering a processable out-signal, a multiplexer coupled to the outputs of said amplifiers, an A/D converter coupled to said multiplexer, and a control unit coupled to sequentially receive outputs from said A/D converter

## Patentansprüche

1. Verfahren zur selektiven In-vivo-Druck- und/oder -Temperaturmessung mit den folgenden Schritten:

   Bereitstellen eines Drucksensors mit zwei Piezowiderstandselementen ($R_a$ $R_p$), wobei die Piezowiderstandselemente mit einer Steuereinheit durch Kabel ($R_{C_{com}}$, $R_{C_a}$, $R_{C_p}$) mit einer Temperaturabhängigkeit gekoppelt sind, die das von den Sensoren bezogene Signal beeinflußt,
   Anregen des Sensors, um ihm zwei verschiedene Signale abzugewinnen, so daß zumindest eine Komponente zumindest eines der Signale den Kabelwiderstand darstellt, und
   **gekennzeichnet durch** den Schritt:

   Verwenden der Größe, die den Kabelwiderstandswert darstellt, um den Einfluß des Kabelwiderstands auf das Temperatur- und/oder Drucksignal zu kompensieren.

2. Verfahren nach Anspruch 1, wobei das erste Piezowiderstandselement ($R_a$) Teil eines Zweigs einer Wheatstoneschen Brücke ist und das zweite Piezowiderstandselement (Rp) Teil eines anderen Zweigs der Wheatstoneschen Brücke ist und wobei die Anregung eine erste Anregung der gesamten Brücke und eine zweite Anregung lediglich des Zweigs der Brücke, die das erste Piezowiderstandselement enthält, aufweist.

3. Verfahren nach Anspruch 2, wobei die Anregung eine an die Brücke angelegte Gleichspannung ist.

4. Verfahren nach Anspruch 3, wobei die zweite Anregung dadurch erfolgt, daß der Zweig, der den zweiten Widerstand enthält, ausgeschaltet wird.

5. Verfahren nach Anspruch 4, wobei die zweite Anregung bei einer niedrigeren Frequenz als die erste Anregung durchgeführt wird.

6. Verfahren nach Anspruch 1, wobei die Anregung eine zusammengesetzte Anregung mit zwei verschiedenen Frequenzen darstellt.

7. Verfahren nach Anspruch 6, wobei die zusammengesetzte Anregung eine Gleichspannungsanregung und eine Wechselspannungsanregung aufweist.

8. Verfahren nach Anspruch 1, wobei die Anregungsspannung bis zu 15 V beträgt.

9. Verfahren nach Anspruch 6, wobei die Wechselspannungskomponente des Signals vom ersten Sensor durch Hochpaßfilterung des Signals vor dessen Einspeisung in einen Verstärker gewonnen wird; das Ausgangssignal des Verstärkers dann in einen Multiplexer und ferner in einen A/D-Umsetzer eingespeist wird, wo das Signal in Digitaldaten umgesetzt wird; die Digitaldaten dann von einem CPU/DSP gelesen werden, der beispielsweise den RMS-(Effektiv-)Wert des Signals berechnet, die Wechselspannungskomponente des Signals vom zweiten Sensor durch Hochpaßfilterung des Signals vor Einspeisung in den Verstärker gewonnen wird, das Ausgangssignal des Verstärkers dann in den Multiplexer und ferner in den A/D-Umsetzer eingespeist wird, wo das Signal in Digitaldaten umgesetzt wird, das Digitalsignal dann von einem CPU/DSP gelesen wird, der auch beispielsweise den RMS-(Effektiv-)Wert des Temperatursignals berechnet, und wobei die Gleichspannungskomponente des Signals vom zweiten Sensor tiefpaßgefiltert wird und das gefilterte Signal in einen zweiten Verstärker eingespeist wird, das Ausgangssignal des zweiten Verstärkers in den Multiplexer und ferner in den A/D-Umsetzer eingespeist wird, wo das Signal in Digitaldaten umgesetzt wird, wobei diese Digitaldaten vom CPU/DSP gelesen werden.

10. Verfahren nach Anspruch 1, wobei der Sensor an einem Führungsdraht angeordnet ist.

11. Verfahren nach Anspruch 1, wobei der Sensor an einem Katheter angeordnet ist.

12. Steuereinheit in Kombination mit einer Führungsdrahtanordnung, wobei die Steuereinheit zur Verarbeitung von Signalen von einem Sensor geeignet ist und die Anordnung dafür angepaßt ist, Druck und/oder Temperatur selektiv in vivo zu messen, wobei die Führungsdrahtanordnung einen Drucksensor und elektrische Leiter ($R_{Ccom}$, $R_{Cp}$ $R_{Ca}$) aufweist, die den Sensor mit der Steuereinheit koppeln, wobei der Sensor zwei Piezowiderstandselemente ($R_a$, $R_p$) hat, wobei die Anordnung parasitäre Widerstände ($R_{Ccom}$, $R_{Cp}$ $R_{Ca}$) in den elektrischen Leitern aufweist, die den Sensor mit der Steuereinheit koppeln, wobei die Steuereinheit aufweist:

   eine Einrichtung zur selektiven Anregung des Drucksensors ($SW_1$), um diesem zwei verschiedene Signale abzugewinnen,
   eine Einrichtung zur selektiven Registrierung einer Komponente eines der Signale, das als Antwort auf eine Anregung des Sensors erzeugt wird, wobei die Komponente proportional zu den parasitären Leiterwiderständen ist, und
   **gekennzeichnet durch**
   eine Einrichtung zur Änderung zumindest eines der verschiedenen Signale **durch** Kompensation des Einflusses der parasitären Widerstände unter Verwendung der Komponente, die den parasitären Widerstandswert darstellt.

13. Steuereinheit nach Anspruch 12, wobei die Anregungseinrichtung eine Gleichspannungsquelle aufweist.

14. Steuereinheit nach Anspruch 13, mit einem Schalter ($SW_1$), der betriebsfähig ist, um die Anregungsspannung für die Passivschaltung bei einer gewählten Frequenz zu beschneiden.

15. Steuereinheit nach Anspruch 14, mit einem ersten und zweiten Differenzverstärker jeweils mit Eingängen zum Empfangen des Temperatursignals und Ausgängen, die ein verarbeitbares Ausgabesignal liefern, einem Multiplexer, der mit den Ausgängen des Verstärkers gekoppelt ist, einem A/D-Umsetzer, der mit dem Multiplexer gekoppelt ist, und einer Steuereinheit, die gekoppelt ist, um Ausgangssignale des A/D-Umsetzers sequentiell zu empfangen.

16. Steuereinheit nach Anspruch 12, wobei die Anregungseinrichtung eine Gleichspannungsquelle und eine Wechselspannungsquelle aufweist, wobei die Anregungsgleich- und die Anregungswechselspannung miteinander überlagert sind.

17. Steuereinheit nach Anspruch 15, mit einem ersten Differenzverstärker mit einer Hochpaßfiltereinrichtung, die mit dessen Eingang gekoppelt ist, wobei der Eingang gekoppelt ist, um eine Hochfrequenzkomponente des Drucksignals zu empfangen, einem zweiten Differenzverstärker mit einer Tiefpaßfiltereinrichtung, die mit dessen Eingang gekoppelt ist, wobei der Eingang gekoppelt ist, um eine Niederfrequenzkomponente des Temperatursignals zu empfangen, und einem dritten Differenzverstärker mit einer Hochpaßfiltereinrichtung, die mit dessen Eingang gekoppelt ist, wobei der Eingang gekoppelt ist, um eine Hochfrequenzkomponente des Temperatursignals zu empfangen, und Ausgängen, die ein verarbeitbares Ausgabesignal liefern, einem Multiplexer, der mit den Ausgängen der Verstärker gekoppelt ist, einem A/D-Umsetzer, der mit dem Multiplexer gekoppelt ist, und einer Steuereinheit, die gekoppelt ist, um Ausgangssignale von dem A/D-Umsetzer sequentiell zu empfangen.

**Revendications**

1. Procédé de mesure sélective de la pression et/ou de la température *in vivo*, comprenant les étapes consistant à :

   fournir un capteur de pression comprenant deux éléments piézorésistifs (Ra, Rp), lesdits éléments piézorésistifs étant couplés à une unité de commande par des câbles (RCcom, RCa, RCp) comprenant une dépendance de la température affectant le signal obtenu à partir desdits capteurs,
   fournir une excitation audit capteur pour produire deux signaux différents à partir de celui-ci, de telle sorte qu'au moins un composant d'au moins l'un desdits signaux soit représentatif de la résistance du câble, et **caractérisé par** l'étape consistant à
   utiliser ladite quantité représentative de la valeur de résistance du câble pour compenser l'influence de ladite résistance du câble sur le au moins un desdits signaux de température et de pression.

2. Procédé selon la revendication 1, dans lequel le premier élément piézorésistif (Ra) fait partie d'une branche d'un pont de Wheatstone, et le second élément piézorésistif (Rp) fait partie d'une autre branche dudit pont de Wheatstone, et dans lequel l'excitation comprend une première excitation du pont entier et une seconde excitation de seulement la branche du pont contenant le premier élément piézorésistif.

3. Procédé selon la revendication 2, dans lequel l'excitation est une tension continue appliquée au pont.

4. Procédé selon la revendication 3, dans lequel la seconde excitation est réalisée en éteignant la branche contenant la seconde résistance.

5. Procédé selon la revendication 4, dans lequel ladite seconde excitation est réalisée à une fréquence inférieure à la première excitation.

6. Procédé selon la revendication 1, dans lequel l'excitation constitue une excitation composite à deux fréquences différentes.

7. Procédé selon la revendication 6, dans lequel ladite excitation composite comprend une excitation de tension continue et une excitation de tension alternative.

8. Procédé selon la revendication 1, dans lequel la tension d'excitation va jusqu'à 15 V.

9. Procédé selon la revendication 6, dans lequel la composante alternative du signal en provenance du premier capteur est obtenue par un filtre passe-haut du signal avant de l'alimenter dans un amplificateur ; la sortie dudit amplificateur est ensuite alimentée dans un multiplexeur et ensuite dans un convertisseur analogique-numérique où le signal est converti en données numériques ; lesdites données numériques sont ensuite lues par une UC/DSP qui calcule par exemple la valeur quadratique moyenne (RMS) du signal, la composante alternative du signal en provenance du second capteur est obtenue par un filtre passe-haut du signal avant de l'alimenter dans ledit amplificateur, la sortie dudit amplificateur est ensuite alimentée dans un multiplexeur et ensuite dans ledit convertisseur analogique-numérique où le signal est converti en données numériques, lesdites données numériques sont ensuite lues par une UC/DSP qui calcule également la valeur quadratique moyenne (RMS) du signal de température, et dans lequel la composante continue du signal en provenance du second capteur est filtrée passe-bas, et ledit signal filtré est alimenté dans un second amplificateur, la sortie dudit second amplificateur étant alimentée dans ledit multiplexeur et également audit convertisseur analogique-numérique où le signal est converti en données numériques, ces données numériques étant lues par UC/DSP.

10. Procédé selon la revendication 1, dans lequel le capteur est monté sur un fil guide.

**11.** Procédé selon la revendication 1, dans lequel le capteur est monté sur un cathéter.

**12.** Unité de commande en combinaison avec un ensemble de fil guide, l'unité de commande étant adaptée au traitement des signaux en provenance d'un capteur, ledit ensemble étant adapté pour mesurer sélectivement la pression et/ou la température *in vivo*, ledit ensemble de fil guide comprenant un capteur de pression et des conducteurs électriques (RCcom, RCa, RCp) couplant ledit capteur à ladite unité de commande, ledit capteur comprenant deux éléments piézorésistifs (Ra, Rp), ledit ensemble comprenant des résistances parasites (RCcom, RCa, RCp) dans lesdits conducteurs électriques couplant ledit capteur à l'unité de commande, l'unité de commande comprenant :

un moyen pour l'excitation sélective dudit capteur de pression ($SW_1$) de façon à produire deux signaux différents à partir de celui-ci,
un moyen pour l'enregistrement sélectif d'une composante de l'un desdits signaux générés en réponse à l'excitation dudit capteur, ladite composante étant proportionnelle auxdites résistances du conducteur parasite, et **caractérisé par**
un moyen pour compenser au moins l'un desdits différents signaux pour l'influence desdites résistances parasites, en utilisant ladite composante représentative de la valeur de résistance parasite.

**13.** Unité de commande selon la revendication 12, dans laquelle le moyen d'excitation comprend une source de tension continue.

**14.** Unité de commande selon la revendication 13, comprenant un commutateur ($SW_1$) utilisable pour permettre de couper la tension d'excitation du circuit passif à une fréquence sélectionnée.

**15.** Unité de commande selon la revendication 14, comprenant un premier et un second amplificateurs instrumentaux comprenant des entrées destinées à recevoir ledit signal de température, respectivement, et des sorties destinées à fournir un signal sortant traitable, un multiplexeur couplé aux sorties desdits amplificateurs, un convertisseur analogique-numérique couplé audit multiplexeur, et une unité de commande couplée pour recevoir séquentiellement les sorties dudit convertisseur analogique-numérique.

**16.** Unité de commande selon la revendication 12, dans lequel le moyen d'excitation comprend une source de tension continue et une source de tension alternative, moyennant quoi les tensions d'excitation continue et alternative sont superposées l'une sur l'autre.

**17.** Unité de commande selon la revendication 15, comprenant un premier amplificateur instrumental comprenant un moyen de filtre passe-haut couplé à son entrée, ladite entrée étant couplée de façon à recevoir une composante haute fréquence dudit signal de pression, un second amplificateur instrumental comprenant un moyen de filtre passe-bas couplé à son entrée, ladite entrée étant couplée de façon à recevoir une composante basse fréquence dudit signal de température, et un troisième amplificateur instrumental comprenant un moyen de filtre passe-haut couplé à son entrée, ladite entrée étant couplée de façon à recevoir une composante haute fréquence dudit signal de température et des sorties fournissant un signal sortant à traiter, un multiplexeur couplé aux sorties desdits amplificateurs, un convertisseur analogique-numérique couplé audit multiplexeur, et une unité de commande couplée pour recevoir séquentiellement les sorties dudit convertisseur analogique-numérique.

Fig. 1a

Fig. 1b

EP 1 055 392 B1

Fig. 2

Fig. 3                    (Prior Art)

Fig. 4

Fig. 5

Fig. 6

Fig. 7

Fig. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

*   WO 9727802 A
*   US 5715827 A
*   US 4554927 A

**Non-patent literature cited in the description**

*   **NICO H J. PIJLS.** Fractional Flow Reserve. *Circulation,* 01 December 1995, vol. 92 (11